# EUROPEAN PATENT APPLICATION

(11) **EP 2 698 116 A1**
(43) Date of publication of application: **19.02.2014**
(21) Application number: 12180658.2
(22) Date of filing: 16.08.2012
(51) Int. Cl.: A61B 10/02

(54) **Biopsy assembly for obtaining a tissue sample**

(71) Applicant: AprioMed AB, 754 50 UPPSALA (SE)
(72) Inventor: Åkerfeldt, Dan, 755 92 Uppsala (SE); Åström, Gunnar, 752 31 Uppsala (SE)
(74) Representative: Brann AB

(57) **Abstract**

The present invention relates to a biopsy assembly (1), for obtaining a tissue sample. The assembly (1) comprises an elongated tube shaped sleeve (2), extending along a longitudinal axis A. The sleeve (2) comprises a first cutting edge arrangement (3) at a distal end (4) of said sleeve (2), said sleeve (2) being provided with an opening (5) at said distal end (4), wherein said opening (5) being in communication with a cavity (6) adapted to receive said tissue sample. The assembly (1) further comprises an elongated drill unit (7), adapted to be removably arranged in said sleeve (2), and extending along a longitudinal axis B, said elongated drill unit (7) comprising a second cutting arrangement (8) arranged at a distal tip (9) of said drill unit (7). The assembly (1) is adapted to be in a first state, during an insertion phase of said assembly (1), in which state said elongated drill unit (7) is arranged in said sleeve (2), and in a second state, during a tissue sampling phase, wherein said elongated drill unit (7) is removed from said sleeve (2). The assembly (1) comprises a fixation member (10), wherein said sleeve (2) and said drill unit (7) are fixated in relation to each other by said fixation member (10), such that in said first state, said first cutting arrangement (3) and said second cutting arrangement (8) are adapted to perform a cooperating cutting action, when said assembly (1) is rotated in a first direction (d₁).

## Description

### Field of the invention

The present invention relates to a biopsy assembly, and in particular to a biopsy assembly for bone marrow biopsy, according to the preamble of the independent claim.

### Background of the invention

Biopsy sampling in bone or sclerotic lesions may be difficult to carry out with the aid of a biopsy needle, e.g. in bone the lesion is often delimited by the hard surface layer of the bone, i.e. the cortical bone tissue. For example in bone marrow biopsy procedures, the biopsy instrument must puncture the cortical bone in order to obtain a sample of the soft tissue, i.e. the marrow, located within the cortical bone.

Today there are several known instruments and procedures to gain access to lesions in bone, and one way is to use a puncture instrument comprising a needle and a sleeve which together works as a drill. For example in US 3628524 A such a biopsy needle is disclosed. The biopsy needle comprises an elongate stylet which is insertable into the needle and has a closed distal end which is positioned in close proximity to and cooperates with the distal cutting edge of the needle. The stylet and needle are releasably interlocked together, whereupon removal of the stylet a biopsy tissue will be collected in the distal end portion of the needle.

Another example is disclosed in EP 2351531 A1, which shows a bone marrow extractor. The bone marrow extractor has an outer needle rod with a case having an upper portion and a lower portion in communication with each other. The case has a front end with a crushing blade and a rear portion with a stopper groove. An inner needle rod which has a main body having a front end with a crushing blade and a rear portion with a connector is inserted into the outer needle rod. The main body of the inner needle rod has an outer surface from which a stopper lever is protruded. The front end of the main body of the inner needle rod is protruded from the front end of the case of the outer needle rod when the stopper lever is fitted into the stopper groove. The inner needle rod and the outer needle rod cooperate with each other to crush bones through a rotation when the head of a driving means coupled to the connector operates. After bone crushing, only the inner needle rod is removed, and the outer needle rod is kept within the human body to suck and extract bone marrow in a syringe-like manner.

In US 20060247653 Al a surgical puncture instrument including a drill and a cutter sleeve is disclosed. The sleeve is shaped to continue the shape of the drill so that the sleeve participates in the drilling operation. This permits the sleeve to enter hard tissue and allows the sleeve to be used for access to inner tissue to, for example, obtain a biopsy with a biopsy needle.

In US 2008/0306405 A1 a trocar comprising a rigid tube wherein may slide a rod with a perforating distal tip, is disclosed. The distal tip zone of the rod forms a perforating drill capable of rotating on its axis while the distal end of the tube is divided into at least two segments with a helical cutting edge. The invention is applicable in particular to bone biopsy.

US 2008/0045965 Al discloses an apparatus and a method to remove biopsy specimens from a bone and/or associated bone marrow using a powered drive and an intraosseous needle set. Each needle set may include a cannula or catheter and an associated trocar stylet with respective tips operable to penetrate a bone and/or bone marrow with minimum trauma to a patient. A stylet is releasably disposed within an associated generally hollow cannula.

The inventors of the present invention has identified a need for an improved biopsy device which provides for a simplified procedure which is less painful procedure for the patient.

Thus, an object of the present invention is to provide a device which provides for an improved and simplified procedure for obtaining a tissue sample, and which provides for a less painful procedure for the patient.

A further object of the present invention is to provide a device which is suitable to introduce, and to obtain a tissue sample with, in both hard and soft tissue.

### Summary of the invention

The above-mentioned objects are achieved by the present invention according to the independent claim.

Preferred embodiments are set forth in the dependent claims.

The biopsy assembly, for obtaining a tissue sample, in accordance with the present invention, comprises an elongated tube shaped sleeve, extending along a longitudinal axis A, and comprising a first cutting edge arrangement at a distal end of said sleeve. The sleeve is provided with an opening at said distal end, wherein said opening is in communication with a cavity adapted to receive said tissue sample. The biopsy assembly further comprises an elongated drill unit, adapted to be removably arranged in said sleeve, and extending along a longitudinal axis B. The elongated drill unit comprises a second cutting arrangement arranged at a distal tip of said drill unit. The assembly is adapted to be in a first state, during an insertion phase of said assembly, in which state said elongated drill unit is arranged in said sleeve, and in a second state, during a tissue sampling phase, wherein said elongated drill unit is removed from said sleeve. The assembly comprises a fixation member, wherein said sleeve and said drill unit are fixated in relation to each other by said fixation member, such that in said first state, said first cutting arrangement and said second cutting arrangement are adapted to perform a cooperating cutting action, when said assembly is rotated in a first direction.

Thus, the sleeve is shaped to continue the shape of the drill unit so that the sleeve participates in the drilling operation.

A further advantage is that the assembly is introduced into hard tissue with a cutting action. Dilation of the tissue is thereby reduced. Thereafter, when the assembly has reached through the hard tissue the drill unit is removed and the sleeve is used to obtain the tissue sample.

### Short description of the appended drawings

Figure 1 shows an elevated view of the biopsy assembly when in a first state, according to one embodiment of the present invention.
Figure 2 shows a side view of the biopsy assembly illustrating that a recess of the sleeve and a flute of the drill unit are essentially aligned in the first state, according to one embodiment of the present invention.
Figure 3 shows a further side view of the biopsy assembly shown in Figure 2, wherein the biopsy assembly is rotated approximately 90° in relation to the view shown in Figure 2.
Figure 4 shows a front view of the biopsy assembly in the first state, according to one embodiment of the present invention.
Figure 5a schematically shows the fixation member provided at a proximal end of the biopsy assembly, according to one embodiment of the present invention,
Figure 5b schematically shows the fixation member, according to one embodiment of the present invention.
Figure 6 shows an elevated view of a portion of the biopsy assembly when in a second state, according to one embodiment of the present invention.
Figure 7 shows a side view of the biopsy assembly, when in the second state, the sleeve being provided with a recess, according to one embodiment of the present invention.
Figure 8 shows the embodiment shown in Figure 7, the biopsy assembly is rotated approximately 90° in relation to the view shown in Figure 6.
Figure 9 shows a longitudinal cross-section of the biopsy assembly when in the second state, according to one embodiment of the present invention.

### Detailed description of preferred embodiments of the invention

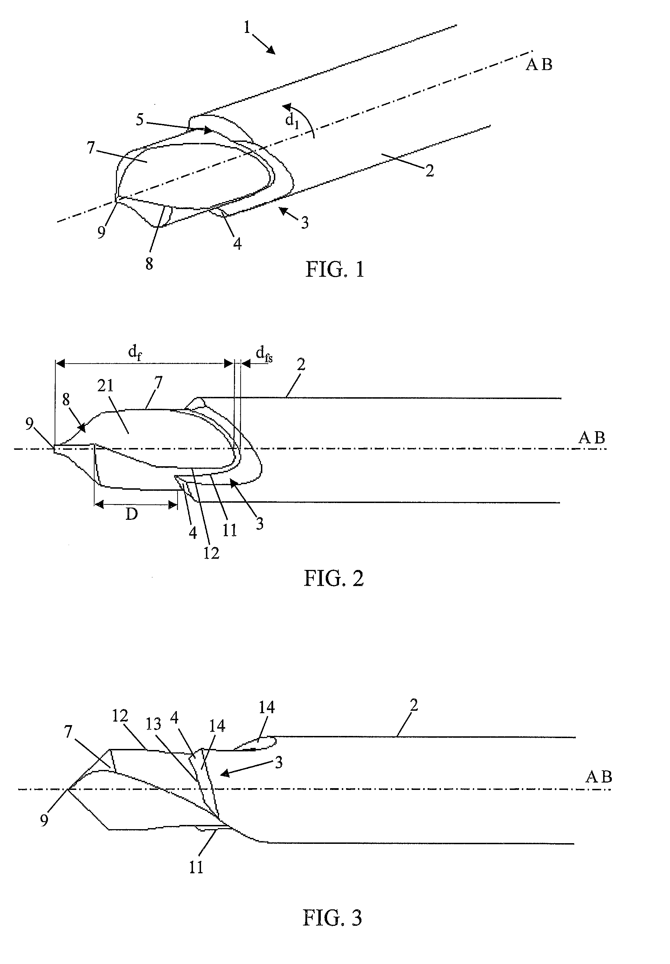

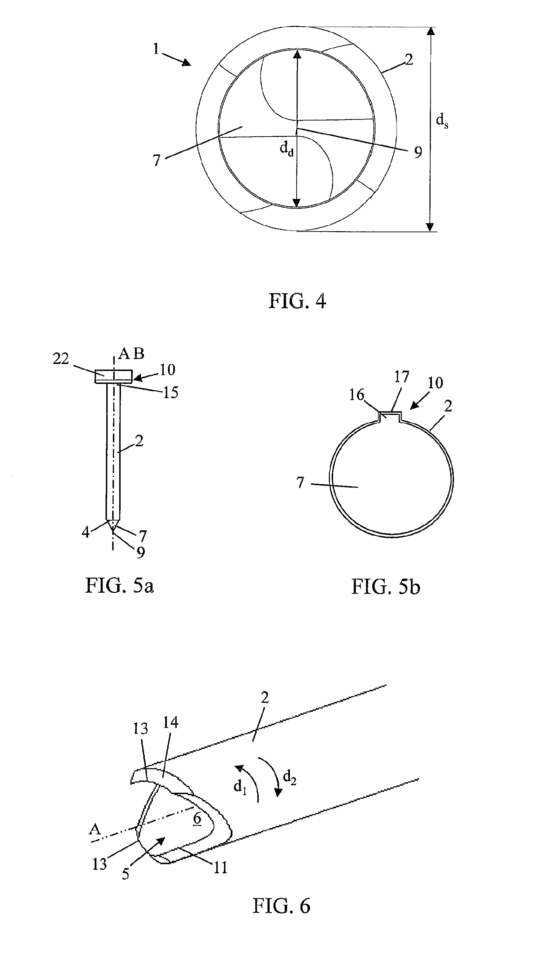

According to the present invention, the biopsy assembly 1 is adapted to be in a first state, during an insertion phase of the assembly 1, and in a second state, during a tissue sampling phase. In the first state the elongated drill unit 7 is arranged in the sleeve 2, as illustrated in Figures 1-5, and in the second state, during a tissue sampling phase, the elongated drill unit 7 is removed from the sleeve 2, as illustrated in Figures 6-9. In the first state, the assembly 1 provides a cutting action during insertion, and in the second state, a tissue sample may be obtained by means of said sleeve 2.

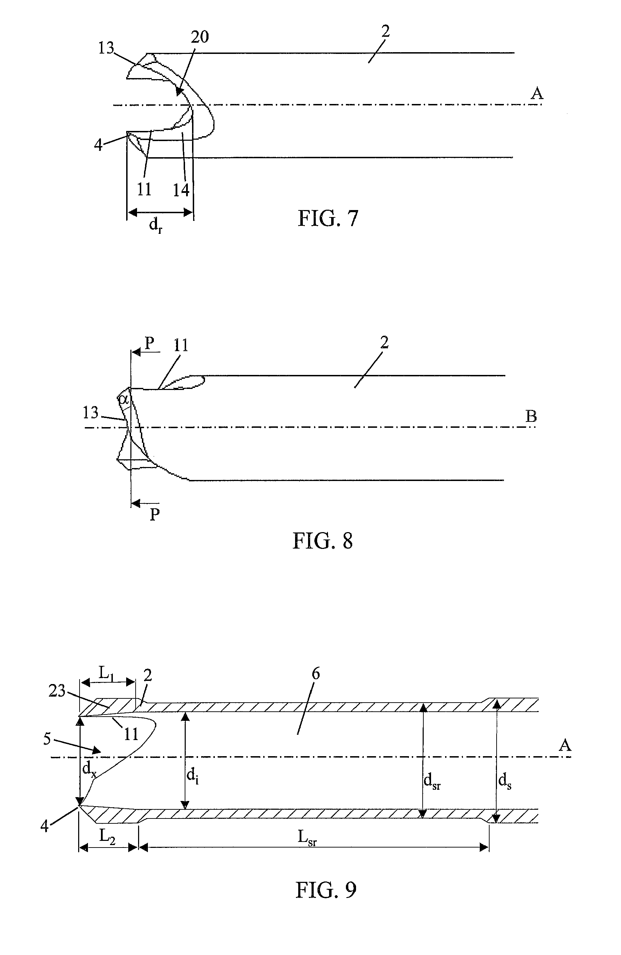

Figure 1 shows the biopsy assembly 1, for obtaining a tissue sample, when in a first state, according to one embodiment of the present invention. The biopsy assembly 1 comprises an elongated tube shaped sleeve 2, extending along a longitudinal axis A. The sleeve 2 comprises a first cutting edge arrangement 3 at a distal end 4 of the sleeve 2, and the sleeve 2 is provided with an opening 5 at the distal end 4. The opening 5 is in communication with a cavity 6 (see e.g. figures 6 and 9) adapted to receive the tissue sample. The biopsy assembly 1 further comprises an elongated drill unit 7, adapted to be removably arranged in the sleeve 2, and extending along a longitudinal axis B. The elongated drill unit 7 comprises a second cutting arrangement 8 arranged at a distal tip 9 of the drill unit 7. As seen in Figure 1, the longitudinal axis A and the longitudinal axis B are aligned in the first state.

Furthermore, the assembly 1 comprises a fixation member 10 (not shown in Figure 1), wherein the sleeve 2 and the drill unit 7 are fixated in relation to each other by the fixation member 10, such that in the first state, the first cutting arrangement 3 and the second cutting arrangement 8 are adapted to perform a cooperating cutting action, when the assembly 1 is rotated in a first direction (d₁). Thus, the sleeve 2 is shaped such that it continues the shape of the drill unit 7 so that the drill unit 7 and the sleeve 2 participates in the drilling operation. Advantageously, the assembly 1 reduces occurrence of dilation of tissue during insertion, and thereby the assembly 1 provides a less painful procedure for the patient.

According to one embodiment, the fixation member 10 is adapted to prevent rotation of the drill unit 7 in relation to the sleeve 2 in the first state. In one embodiment, the fixation member 10 is adapted to assure a predetermined longitudinal position of the drill unit 7 in relation to the sleeve 2 in the first state.

According to one embodiment, as shown in Figure 2, the distal tip 9 of the drill unit 7 projects a predetermined distance D from the distal end 4 of the sleeve 2 in the predetermined longitudinal position. The distance D is approximately 0-5 mm. The first cutting edge arrangement 3 of the sleeve 2 comprises a first cutting edge 11, and the second cutting arrangement 8 of the drill unit 7 comprises a longitudinal cutting edge 12 extending at least partly along the drill unit 7, wherein the first cutting edge 11 and the longitudinal cutting edge 12 are adapted to be aligned, or essentially aligned, in the first state.

In the embodiment shown in Figure 2, the elongated drill unit 7 comprises at least one flute 21 extending helically, from the distal tip 9, along a distance d_{f} of the elongated drill unit 7.

According to one embodiment, the first cutting edge 11 is arranged to extend essentially in the same direction as the longitudinal axis A, as shown in Figures 1-2.

According to one embodiment, as illustrated in Figure 3, the first cutting edge arrangement 3 of the sleeve 2 comprises a second cutting edge 13. As seen in Figure 3, the sleeve 2 is provided with a chamfering 14 at the distal end 4, preferably at said first cutting edge 11 and at said second cutting edge 13. The chamfering 14 provides for an improved chip removal during the insertion phase. The chamfering 14 also prevents soft tissue from being damaged during insertion of the biopsy assembly 1.

Figure 4 shows a front view of the biopsy assembly 1 when being in the first state. The drill unit 7 is concentric and the diameter d_{d} of the drill unit is between 1-5 mm. The inner diameter of the sleeve 2 is adapted to the diameter d_{d} of the drill unit 7 such that the drill unit 7 may be introduced into and arranged in said sleeve 2. The inner diameter dᵢ of the sleeve 2 is preferably between 0,01-0,2 mm larger than the diameter d_{d} of the drill unit 7. The outer diameter dₛ of the sleeve 2 is preferably between 1,5-6 mm. The outer diameter dₛ may be decreasing towards the distal end 4, in a distal end portion 23 of the sleeve 2.

In the embodiment shown in Figure 5a, the fixation member 10 is provided essentially at a proximal end 15 of the biopsy assembly 1. In Figure 5a, the biopsy assembly 1 is in the first state, thus the drill unit 7 is arranged in the sleeve 2, and the distal tip 9 of the drill unit 7 projects from the distal end 4 of the sleeve 2. The biopsy assembly 1 is further provided with a handle 22 which facilitates rotation of the assembly 1.

According to one embodiment, as illustrated in Figure 5b, the fixation member 10 comprises an indentation 16 and a mating recess 17. Preferably, the indentation 16 is provided at, or interconnected with said drill unit 7, and said mating recess 17 is provided at, or interconnected with, said sleeve 2. The indentation 16 and the mating recess 17 assures longitudinal position and prevents rotation of the drill unit 7 and sleeve 2 in relation to each other. However, the fixation member 10 may comprise any other suitable means for fixating said sleeve 2 and said drill unit 7 in relation to each other, such as e.g. pin and pin hole, snap lock or threads.

Figure 6 illustrates the biopsy assembly 1 in the second state. The drill unit 7 has been removed from the sleeve 2, such that the cavity 6 within the drill unit 7 may receive the tissue sample via the opening 5. According to one embodiment, as shown in Figure 6, the first cutting edge 11 is adapted to perform a cutting action when the sleeve 2 is rotated in a first direction d₁, and the second cutting edge 13 is adapted to perform a cutting action when the sleeve 2 is rotated in a second direction d₂, opposite the first direction d₁. The shape of the first cutting edge 11 is advantageous when obtaining a tissue sample in bone or sclerotic lesions, i.e. in relatively hard tissue. Consequently, when obtaining a sample in bone or sclerotic lesions, the sleeve 2 is preferably rotated in the first direction d₁. The shape of the second cutting edge 13 is advantageous when obtaining a sample from relatively soft tissue. Therefore, when obtaining a sample in soft tissue, the sleeve 2 is preferably rotated in the second direction d₂. The first direction d₁ may be clockwise direction and the second direction d₂ may be counter-clockwise direction.

According to one embodiment, as shown in Figure 7, the first cutting edge 11 is arranged in a recess 20 extending from the distal end 5 of the sleeve 2 at least partly along the sleeve 2. The recess 20 extends a distance dᵣ from the distal end 4 along the sleeve 2. Preferably, the distance dᵣ is between 1-10 mm. In the first state, as shown in Figures 1-2, the recess 20 is arranged to be aligned, or essentially aligned, with the flute 21. As illustrated in Figure 2, the distance d_{fs}, between the most proximal part of the flute 21 and the first cutting edge 11 extending along the recess 20, is preferably 0-3 mm.

In one embodiment, as shown in Figure 8, the second cutting edge 13 comprises an inclined cutting edge, the inclined cutting edge extends in an angle α with respect to a plane P perpendicular to the longitudinal axis A. Preferably, the angle a is approximately 5-30 degrees. The inclined cutting edge 13 is advantageous in that it facilitates cutting in soft tissue when the sleeve 2 is rotated in the second direction d₂.

Figure 9 shows a longitudinal cross-section of the sleeve 2, according to one embodiment of the present invention. The sleeve 2 is tubular and comprises a cavity 6 adapted to receive the tissue sample via the opening 5, when the assembly 1 is in the second state. The inner diameter dᵢ of the sleeve 2, which corresponds to the diameter of the cavity 6, may be decreasing towards the distal end 4. Thus, the cavity 6 then tapers towards the distal end 4. Preferably, the inner diameter dᵢ of the sleeve 2 is decreasing in a distal end portion 23 of the sleeve 2, essentially at the distal end 4 of the sleeve 2. The length L₁ of the distal end portion 23, having a decreasing inner diameter dᵢ, is approximately 1-10 mm. A decreasing inner diameter dᵢ is advantageous in that the friction is reduced, which facilitates forcing the tissue sample into the cavity 6. Preferably, the difference between the inner diameter dᵢ and the diameter dₓ at the distal end 4 of the sleeve 2 is approximately 0,1 - 0,5 mm.

According to the embodiment shown in Figure 9, the sleeve 2 is provided with a reduced outer diameter dₛᵣ along a length Lₛᵣ of the sleeve 2. The length Lₛᵣ is approximately between 5-40 mm. The reduced outer diameter dₛᵣ is less than the outer diameter dₛ, and the difference between the outer diameter dₛ of the sleeve 2 and the reduced outer diameter dₛᵣ is approximately 0,05 - 0,3 mm. A reduced outer diameter along part of the sleeve is advantageous in that it reduces friction during insertion of the assembly 1. Preferably, the length L₂ between the distal end 4 of the sleeve 2 and the most distal portion of the portion of the sleeve 2 having a reduced outer diameter dₛᵣ is between 1-10 mm.

The present invention is not limited to the above-described preferred embodiments. Various alternatives, modifications and equivalents may be used. Therefore, the above embodiments should not be taken as limiting the scope of the invention, which is defined by the appending claims.

## Claims

1. Biopsy assembly (1), for obtaining a tissue sample, said assembly (1) comprising:
- an elongated tube shaped sleeve (2), extending along a longitudinal axis A, and comprising a first cutting edge arrangement (3) at a distal end (4) of said sleeve (2), said sleeve (2) being provided with an opening (5) at said distal end (4), wherein said opening (5) being in communication with a cavity (6) adapted to receive said tissue sample;
- an elongated drill unit (7), adapted to be removably arranged in said sleeve (2), and extending along a longitudinal axis B, said elongated drill unit (7) comprising a second cutting arrangement (8) arranged at a distal tip (9) of said drill unit (7);
wherein said assembly (1) is adapted to be in a first state, during an insertion phase of said assembly (1), in which state said elongated drill unit (7) is arranged in said sleeve (2), and in a second state, during a tissue sampling phase, wherein said elongated drill unit (7) is removed from said sleeve (2),
**characterized in that** said assembly (1) comprises a fixation member (10), wherein said sleeve (2) and said drill unit (7) are fixated in relation to each other by said fixation member (10), such that in said first state, said first cutting arrangement (3) and said second cutting arrangement (8) are adapted to perform a cooperating cutting action, when said assembly (1) is rotated in a first direction (d₁).

2. Biopsy assembly according to claim 1, wherein said fixation member (10) is adapted to prevent rotation of said drill unit (7) in relation to said sleeve (2) in said first state.

3. Biopsy assembly according to any of claims 1-2, wherein said fixation member (10) is adapted to assure a predetermined longitudinal position of said drill unit (7) in relation to said sleeve (2) in said first state.

4. Biopsy assembly according to claim 3, wherein said distal tip (9) of said drill unit (7) projects a predetermined distance D from said distal end (4) of said sleeve (2) in said predetermined longitudinal position.

5. Biopsy assembly according to any of claims 1-4, wherein said first cutting edge arrangement (3) of said sleeve (2) comprises a first cutting edge (11), and said second cutting arrangement (8) of said drill unit (7) comprises a longitudinal cutting edge (12) extending at least partly along said drill unit (7), wherein said first cutting edge (11) and said longitudinal cutting edge (12) are adapted to be essentially aligned, in said first state.

6. Biopsy assembly according to any of claims 1-5, wherein said first cutting edge arrangement (3) of said sleeve (2) comprises a second cutting edge (13).

7. Biopsy assembly according to claim 6, wherein, in said second state, said first cutting edge (11) is adapted to perform a cutting action when said sleeve (2) is rotated in a first direction (d₁), and wherein said second cutting edge (13) is adapted to perform a cutting action when said sleeve (2) is rotated in a second direction (d₂), opposite said first direction (d₁).

8. Biopsy assembly according to any of claims 5-7, wherein said first cutting edge (11) is arranged in a recess (20) extending from said distal end (4) at least partly along said sleeve (2).

9. Biopsy assembly according to any of claims 1-8, wherein said elongated drill unit (7) comprises at least one flute (21) extending helically, from said distal tip (9), along a distance d_{f} of said elongated drill unit (7).

10. Biopsy assembly according to claim 9 when dependent on claim 8, wherein said recess (20) is arranged to be aligned with said flute (21) in said first state.

11. Biopsy assembly according to any of claims 5-10, wherein said first cutting edge (11) is arranged to extend essentially in the same direction as said longitudinal axis A.

12. Biopsy assembly according to any of claims 6-11, wherein said second cutting edge (13) comprises an inclined cutting edge, said inclined cutting edge extends in an angle α with respect to a plane P perpendicular to said longitudinal axis A.

13. Biopsy assembly according to claim 12, wherein said angle α is approximately 5-30 degrees.

14. Biopsy assembly according to claim 4, wherein said predetermined distance D is approximately 0-5 mm.

15. Biopsy assembly according to any of the preceding claims, wherein said fixation member (10) is arranged essentially at a proximal end (15) of said assembly (1).

16. Biopsy assembly according to any of the preceding claims, wherein said sleeve (2) has an inner diameter dᵢ, and wherein said inner diameter dᵢ is decreasing, towards said distal end (4), in a distal end portion (23) of said sleeve (2).

17. Biopsy assembly according to any of the preceding claims, wherein said sleeve (2) has an outer diameter dₛ, and wherein said outer diameter dₛ is decreasing, towards said distal end (4), in a distal end portion (23) of said sleeve (2).

18. Biopsy assembly according to any of the preceding claims, wherein said sleeve (2) is provided with a reduced outer diameter dₛᵣ along a length Lₛᵣ of said sleeve (2).
